# EUROPEAN PATENT SPECIFICATION

(11) **EP 3 844 769 B1**
(45) Date of publication and mention of the grant of the patent: **19.03.2025**
(21) Application number: 19755913.1
(22) Date of filing: 19.08.2019
(51) Int. Cl.: G16H 30/40

(54) **GENERATING METADATA FOR TRAINED MODEL**
ERZEUGEN VON METADATEN FÜR TRAINIERTE MODELLE
GÉNÉRATION DE MÉTADONNÉES POUR MODÈLE FORMÉ

(30) Priority: 27.08.2018 EP 18190931
(43) Date of publication of application: 07.07.2021
(73) Proprietor: Koninklijke Philips N.V., 5656 AG Eindhoven (NL)
(72) Inventor: BAKKER, Bart, Jacob, 5656 AE Eindhoven (NL); MAVROEIDIS, Dimitrios, 5656 AE Eindhoven (NL); TRAJANOVSKI, Stojan, 5656 AE Eindhoven (NL)
(74) Representative: Philips Intellectual Property & Standards
(86) International application number: PCT/EP2019/072157
(87) International publication number: WO 2020/043530

(56) References cited:
- ZIHANG DAI ET AL: "Good Semi-supervised Learning that Requires a Bad GAN", 31ST CONFERENCE ON NEURAL INFORMATION PROCESSING SYSTEMS (NIPS 2017), 3 November 2017 (2017-11-03), XP055556220, Retrieved from the Internet <URL:https://arxiv.org/pdf/1705.09783.pdf> [retrieved on 20190213]
- JONGHOON KIM ET AL: "Prospects of deep learning for medical imaging", PRECISION AND FUTURE MEDICINE, vol. 2, no. 2, 30 June 2018 (2018-06-30), pages 37 - 52, XP055556475, ISSN: 2508-7940, DOI: 10.23838/pfm.2018.00030
- IAN J GOODFELLOW ET AL: "Generative Adversarial Nets", CORNELL UNIVERSITY LIBRARY - ARXIV.ORG E-PRINT ARCHIVE, 10 June 2014 (2014-06-10), pages 1 - 9, XP055556208, Retrieved from the Internet <URL:https://arxiv.org/pdf/1406.2661.pdf> [retrieved on 20190213]
- ELI GIBSON ET AL: "NiftyNet: a deep-learning platform for medical imaging", COMPUTER METHODS AND PROGRAMS IN BIOMEDICINE., vol. 158, 1 May 2018 (2018-05-01), NL, pages 113 - 122, XP055557277, ISSN: 0169-2607, DOI: 10.1016/j.cmpb.2018.01.025
- YANG YU ET AL: "Open Category Classification by Adversarial Sample Generation", PROCEEDINGS OF THE TWENTY-SIXTH INTERNATIONAL JOINT CONFERENCE ON ARTIFICIAL INTELLIGENCE, 1 August 2017 (2017-08-01), California, pages 3357 - 3363, XP055556209, ISBN: 978-0-9992411-0-3, DOI: 10.24963/ijcai.2017/469

## Description

### FIELD OF THE INVENTION

The invention relates to a system and a computer-implemented method for processing a trained model, such as a trained neural network, which is trained on training data. The invention further relates to a system and a computer-implemented method for using the trained model with input data, for example, for classification of the input data. The invention further relates to a computer-readable medium comprising instructions to perform either computer-implemented method.

### BACKGROUND OF THE INVENTION

Machine learning is playing an increasingly important role in various domains and for various purposes. For example, in the medical domain, machine learning techniques such as deep learning have been found to be very suitable for classification and segmentation of image content from modalities including CT, X-ray, digital pathology and MRI. As is known per se, such machine learning may be used to train a model, such as a neural network, using training data as input. After such training, the trained model may be applied to new input data, e.g., to obtain a prediction from or classification of the new data. In a specific example, the trained model may be trained on labeled medical images and, during later use, applied to a medical image to obtain a classification or segmentation of an anatomical structure in the medical image. Various other uses of trained models are known.

A trained model is typically optimized and validated on a specific dataset. For example, in the example of medical image classification, a model may be trained on images and labels (or other classifications/annotations) from a particular modality. The trained model performance may be expected by users to be consistently accurate. However, this may require the input data, e.g., new images, to conform to the same or similar characteristics as the training data. In practice, it is not guaranteed that a trained model is used under specified conditions (also referred to 'in specification' or 'in-spec' use) as it may rather be used 'out-of-spec'.

For example, a user may apply a trained model to images which were acquired using a different image acquisition apparatus ('scanner') than was used to acquire the training images. Another example is that the settings of the scanner may change, image quality may degrade because of scratches on the lens or a defect in the scanning beam, the radiation dose may be different than required, etc.

### PRIOR ART

"Open Category Classification by Adversarial Sample Generation", by Yu et al., PROCEEDINGS OF THE TWENTY-SIXTH INTERNATIONAL JOINT CONFERENCE ON ARTIFICIAL INTELLIGENCE, 1 August 2017 (2017-08-01), pages 3357-3363, XP055556209,California DOI: 10.24963/ijcai.2017/4691SBN: 978-0-9992411-0-3, relates to a modular deep learning pipeline for medical image processing including segmentation, regression, image generation and representation learning.

### SUMMARY OF THE INVENTION

The invention is defined by the appended claims.

It may be desirable to facilitate in-spec use of a trained model.

The following aspects of the invention may involve generating metadata which encodes a numerical characteristic of the training data of a trained model, and using the metadata to determine conformance of input data of the trained model to the numerical characteristics of the training data. Accordingly, the system applying the trained model to the input data may, for example, warn a user that the use of the trained model with the input data is considered out of specification ('out-of-spec'), or may decline to apply the trained model to the input data, etc.

In accordance with a first aspect of the invention, a system is provided for processing a trained model. The system comprises:
a data interface for accessing:
   - model data representing a trained model, and
   - training data on which the trained model is trained;
a processor subsystem configured to:
   - characterize the training data by:
      applying the trained model to the training data to obtain intermediate output of the trained model, and
      determining the numerical characteristic based on the intermediate output of the trained model;
   - encode the numerical characteristic as metadata; and
   - associate the metadata with the model data to enable an entity applying the trained model to input data and thereby obtaining further intermediate output of the trained model to determine whether the input data conforms to the numerical characteristic of the training data of the trained model based on the further intermediate output.

In accordance with a further aspect of the invention, a computer-implemented method is provided for processing a trained model, comprising:
accessing:
   - model data representing a trained model, and
   - training data on which the trained model is trained;
characterizing the training data by:
   - applying the trained model to the training data to obtain intermediate output of the trained model, and
   - determining the numerical characteristic based on the intermediate output of the trained model;
encoding the numerical characteristic as metadata; and
associating the metadata with the model data to enable an entity applying the trained model to input data and thereby obtaining further intermediate output of the trained model to determine whether the input data conforms to the numerical characteristic of the training data of the trained model based on the further intermediate output.

In accordance with a further aspect of the invention, a system is provided for using a trained model. The system comprises:
a data interface for accessing:
   - model data representing a trained model having been trained on training data,
   - metadata associated with the model data and comprising a numerical characteristic, wherein the numerical characteristic is determined based on an intermediate output of the trained model when applied to the training data, and
   - input data to which the trained model is to be applied;
a processor subsystem configured to:
   - apply the trained model to the input data to obtain a further intermediate output of the trained model;
   - determine whether the input data conforms to the numerical characteristic of the training data of the trained model based on the further intermediate output; and
   - if the input data is determined not to conform to the numerical characteristic, generate an output signal indicative of said non-conformance.

In accordance with a further aspect of the invention, a computer-implemented method is provided for using a trained model, comprising:
accessing:
   - model data representing a trained model having been trained on training data,
   - metadata associated with the model data and comprising a numerical characteristic, wherein the numerical characteristic is determined based on an intermediate output of the trained model when applied to the training data, and
   - input data to which the trained model is to be applied;
applying the trained model to the input data to obtain a further intermediate output of the trained model;
determining whether the input data conforms to the numerical characteristic of the training data of the trained model based on the further intermediate output; and
if the input data is determined not to conform to the numerical characteristic, generating an output signal indicative of said non-conformance.

In accordance with a further aspect of the invention, a computer-readable medium is provided comprising transitory or non-transitory data representing instructions arranged to cause a processor system to perform either or both computer-implemented methods.

The above measures involve accessing a trained model, such as a trained neural network, and accessing training data on which the trained model is trained. The trained model may be applied to the training data in a manner as if the training data were input data to be processed by the trained model. For example, if the trained model is trained for image classification, the training data may be used as input to the trained model to perform image classification on the training data.

The above measures further involve determining a numerical characteristic from an intermediate output of the trained model. Here, the term 'intermediate output' is to be understood as follows: the trained model processes input data to generate output data, e.g., a classification, a probability, etc. To generate the output data, the trained model generates intermediate output data, being an internal precursor to the output data. As a non-limiting example, in the case of a trained neural network, such internal data may be activation values of hidden units of the trained neural network when applied to the input data. Other types of intermediate output are equally conceivable and dependent on the type of trained model. For example, if the trained model is a trained capsule network, the intermediate output may be one or more activity vectors of capsules. Similar examples exist for other models which are trainable by machine learning, e.g. latent variables in graphical models, such as Bayesian networks, etc.

The intermediate output may effectively represent an internal and intermediate characterization of the input data by the trained model, and when the input data is the training data, an intermediate characterization of the training data. This intermediate characterization may be made tangible by obtaining a numerical characteristic of the intermediate output. Thereby, a numerical characterization of the training data is obtained, albeit in an indirect rather than direct manner. Accordingly, the numerical characteristic is in the above and following also referred to as a numerical characteristic "of" the training data, even if it is not directly calculated from the training data but indirectly from the intermediate output.

The inventors have considered that such a numerical characteristic of the intermediate output of the trained model may be more suited for characterizing the training data than the training data itself. Namely, the training data may have properties which may make it less suitable for such characterization. For example, the training data may be large in terms of data size, which may make it computationally complex to calculate a descriptive numerical characteristic, and/or it may be difficult to manually (heuristically) or otherwise determine a concise characterization of the training data. The trained model, on the other hand, is typically trained to obtain such a concise characterization, e.g., a classification, probability, etc. However, while the output data itself (e.g., the classification or probability) is typically insufficiently descriptive across the range of possible input data, the intermediate output is well-suited to provide such a characterization.

The numerical characteristic may be encoded as metadata, and the metadata may be associated with the trained model, or more specifically with the model data of the trained model, in any suitable manner, e.g., by including the numerical characteristic in the model data itself, e.g., as a file header, XML element, etc., or providing the metadata as a separate file, or in any other manner.

At the application side, e.g., when using the trained model on non-training input data, the metadata may be used to determine whether current input data of the trained model conforms to the characteristic of the training data, and thus whether the use of the trained model with the current input data represents an 'in-spec' use or an 'out-of-spec' use. Namely, the intermediate output of the trained model may be compared or in another way validated against the numerical characteristic encoded in the metadata to determine conformance or non-conformance. If non-conformance is detected, this may be used to, e.g., warn the user, refrain from outputting the classification by the trained model, etc.

Optionally, the trained model is a trained neural network, and the intermediate output comprises activation values of a subset of hidden units of the trained neural network. The activation values of hidden units may be well-suited for determining a numerical characteristic which is descriptive of the training data. A selection of such activation values may be used. In some examples, the trained neural network may be a trained deep neural network, e.g., having several layers of hidden units. The selection of activation values may correspond to a selection of select parts of select layers of the neural network.

Optionally, the training data comprises multiple training data objects, and the processor subsystem is configured to:
- apply the trained model to individual ones of the multiple training data objects to obtain multiple sets of activation values; and
- determine the numerical characteristic of the training data as a probability distribution of the multiple sets of activation values.

The training data may comprise multiple individual training data objects, e.g., multiple images, audio recordings, etc. In general, such training data objects may be characterized by a probability distribution. However, for many data types, obtaining a probability distribution directly from the training data itself is either infeasible or at least computationally complex, as the training data may be quite large (e.g., many high-resolution images), the probability distribution may be too complex to easily characterize, etc. Instead, a probability distribution of the activation values of a selection of hidden units may be generated, e.g., from the numerical outputs of selected parts of selected layers of a deep neural network. This may be less computationally complex as the number of hidden units may be significantly fewer than the number of data elements in each training data object.

Optionally, the processor subsystem is configured to:
- obtain out-of-spec data comprising multiple out-of-spec data objects which are different from, and have characteristics which do not conform to the characteristics of, the multiple training data objects;
- apply the trained neural network to individual ones of the multiple out-of-spec data objects to obtain further multiple sets of activation values;
- and select the subset of hidden units to establish a difference, or to increase or maximize the difference, between a) the probability distribution of the multiple sets of activation values and b) a probability distribution of the further multiple sets of activation values.

It may be desirable to select a specific subset of the hidden units which yields a distinct difference in probability distribution when calculating the probability distribution from the training data or from out-of-spec data. Such out-of-spec data may be considered, e.g., by manual assessment or by an automated metric, data which, when used as input to the trained model, represents an out-of-spec use of the trained model since the characteristics of the out-of-spec data do not conform to the characteristics of the training data, or at least not to a sufficient degree. In particular, the subset may be selected such that a sharp discrimination is obtained between what is considered to be in-spec data and out-of-spec data. For that purpose, out-of-spec data may be obtained which comprises multiple out-of-spec data objects which are different from the multiple training data objects. For example, in case of images, the out-of-spec data may comprise a set of images which have not been used in the training of the trained model and which are considered to represent out-of-spec input data for the trained model. The subset of hidden units, from which the probability distribution is obtained, may be selected to maximize the difference between the training data and the out-of-spec data, or at least to increase the difference compared to, e.g., a random selection or a selection of all hidden units.

For example, the processor subsystem may be configured to select the subset of hidden units by a combinatorial optimization method which optimizes the difference between a) the probability distribution of the multiple sets of activation values and b) the probability distribution of the further multiple sets of activation values, as a function of selected hidden units. The difference may, for example, be a Kullback-Leibler divergence measure, a cross entropy measure, or a mutual information measure, which are known per se.

Optionally, the processor subsystem is configured to:
- use a generator part of a generative adversarial network to generate negative samples on the basis of the training data;
- generate the out-of-spec data from the negative samples.

The generator part of a generative adversarial network (GAN) may be used to create the out-of-spec data in an automated manner, e.g., without manual selection or manual generation of the out-of-spec data, or in combination with a manual selection or manual generation of the out-of-spec data.

Optionally, the processor subsystem is configured to generate the model data by training a model using the training data, thereby obtaining the trained model. The system generating the metadata may in some embodiments be the same system as used for training the trained model. This may be convenient since the system already has access to the training data.

Optionally, the training data comprises multiple images, and the trained model is configured for image classification or image segmentation.

Optionally, the system for using the trained model further comprises an output interface for outputting the output signal to a rendering device for rendering the output signal in a sensory perceptible manner to a user. For example, the system may generate a warning message on a display.

Optionally, the trained model is a trained neural network, the numerical characteristic is a probability distribution obtained from multiple sets of activation values of a subset of hidden units of the trained neural network, the multiple sets of activation values are obtained by applying the trained model to the training data, the further intermediate output of the trained model comprises a further set of activation values of the subset of hidden units, and the processor subsystem of the system for using the trained model is configured to:
- determine a probability of the further set of activation values based on the probability distribution; and
- determine whether the input data conforms to the numerical characteristic of the training data of the trained model as a function of the probability.

The above may represent a specific example of how the system applying the trained model may determine whether the input data conforms to the numerical characteristic of the training data of the trained model.

Modifications and variations of any computer-implemented method and/or any computer program product, which correspond to the described modifications and variations of a corresponding system, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for processing a trained model to generate metadata for the trained model, the metadata encoding a numerical characteristic of the training data on which the trained model is trained;
Fig. 2 shows a system for using the trained model with input data and for using the metadata to determine whether the input data conforms to the numerical characteristic of the training data of the trained model;
Fig. 3 shows a detailed example of how a numerical characteristic may be determined for a trained neural network, being in this example a probability function of activation values of a subset of hidden units of said network;
Fig. 4 shows a method of processing a trained model to generate metadata which encodes a numerical characteristic of the model's training data;
Fig. 5 shows a method of using the trained model with input data and for using the metadata to determine whether the input data conforms to the numerical characteristic of the training data of the trained model; and
Fig. 6 shows a computer-readable medium comprising data.

It should be noted that the figures are purely diagrammatic and not drawn to scale. In the figures, elements which correspond to elements already described may have the same reference numerals.

### List of abbreviations

- DL: Deep Learning
- DNN: Deep Neural Network
- GAN: Generative Adversarial Network
- GenAl: Genetic Algorithm
- KL: Kullback-Leibler

### List of reference numbers

The following list of reference numbers is provided for facilitating the interpretation of the drawings and shall not be construed as limiting the claims.
- 020, 022data: storage
- 030: training data
- 040: input data
- 050: model data
- 060: metadata

- 100: system for processing trained model
- 120: data interface
- 122, 124: data communication
- 140: processor subsystem
- 200: system for using trained model
- 220: data interface
- 222, 224: data communication
- 240: processor subsystem
- 242: output signal
- 260: display output interface
- 262: display data
- 280: display
- 282: warning dialog box

- 300: training data
- 310: generative adversarial network
- 320: trained model
- 330: probability distribution (in-spec)
- 335: probability distribution (out-of-spec)
- 340: difference measure (Kullback-Leibler divergence)
- 350: combinatorial optimization method (genetic algorithm)
- 360: activation values of subset of hidden units, variance

- 400: method of processing trained model
- 410: accessing model data, training data
- 420: characterizing training data
- 430: applying trained model to training data
- 440: determining numerical characteristic
- 450: encoding numerical characteristic as metadata
- 460: associating metadata with model data

- 500: method of using trained model
- 510: accessing model data, metadata, input data
- 520: applying trained model to input data
- 530: determining conformance of input data
- 540: generating output signal indicative of non-conformance
- 600: computer-readable medium
- 610: non-transitory data

### DETAILED DESCRIPTION OF EMBODIMENTS

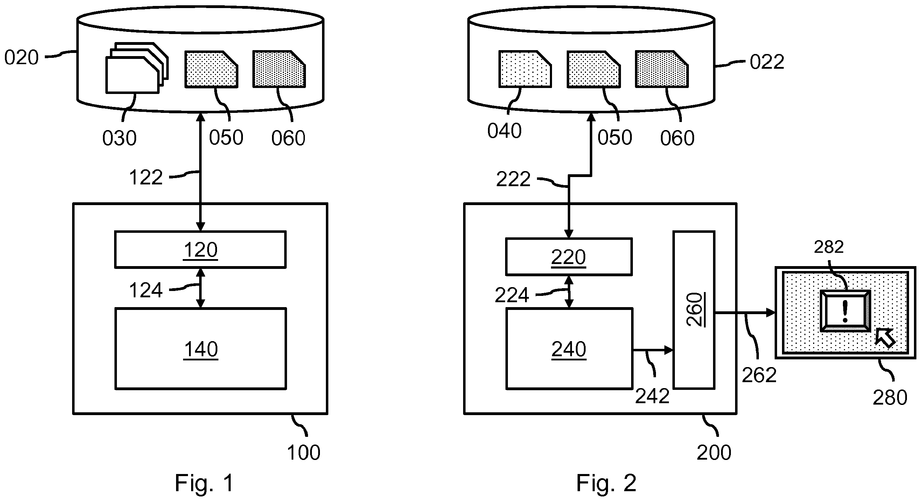

Fig. 1 shows a system 100 for processing a trained model to generate metadata for the trained model which encodes a numerical characteristic of the training data on which the trained model is trained. The system 100 may comprise a data interface 120 and a processor subsystem 140 which may internally communicate via data communication 124. The processor subsystem 140 may be configured to, during operation of the system 100 and using the data interface 120, access model data 050 representing a trained model, and access training data 030 on which the trained model is trained. For example, as shown in Fig. 1, the data interface 120 may provide access 122 to an external data storage 020 which may comprise said data 030, 050. Alternatively, the data 030, 050 may be accessed from an internal data storage which is part of the system 100. Alternatively, the data 030, 050 may be received via a network from another entity. In general, the data interface 120 may take various forms, such as a network interface to a local or wide area network, e.g., the Internet, a storage interface to an internal or external data storage, etc. The data storage 020 may take any known and suitable form.

The processor subsystem 140 may be further configured to, during operation of the system 100, characterize the training data by applying the trained model to the training data to obtain intermediate output of the trained model, and determining a numerical characteristic based on the intermediate output of the trained model. The processor subsystem 140 may encode the numerical characteristic as metadata 060, and associate the metadata 060 with the model data 050 to enable an entity applying the trained model to input data to determine whether the input data conforms to the numerical characteristic of the training data of the trained model. An example of such an entity is the system 200 of Fig. 2.

The metadata 060 may be stored by the system 100 in the data storage 020 or elsewhere, sent via a network, etc. In general, the metadata 060 may be stored in a same data container as the training data 050, for example in a same file(s), but may also be provided as separate metadata 060 which is associated with the training data 050. For example, in some embodiments, the training data 050 may link to the metadata 060, e.g., by containing an URL at which the metadata 060 is accessible, or the metadata 060 may link to the training data 050. Various other means of association are equally conceivable and within reach of the skilled person.

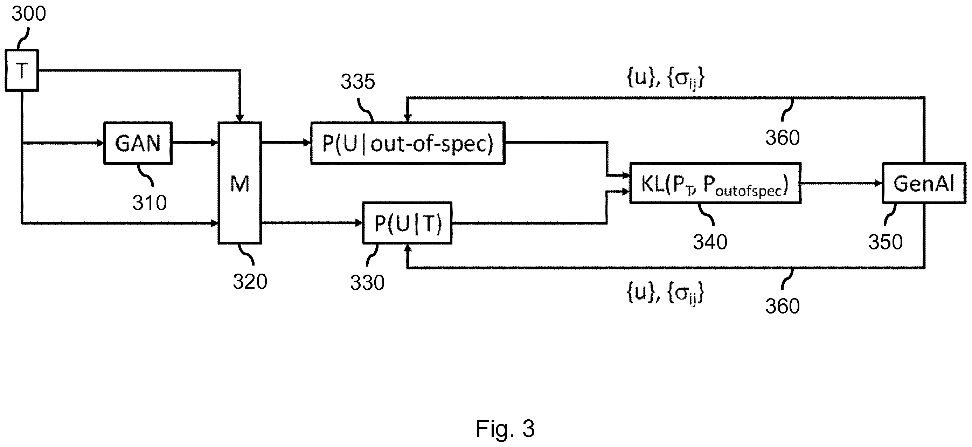

Various details and aspects of the operation of the system 100 will be further elucidated with reference to Fig. 3, including optional aspects thereof.

In general, the system 100 may be embodied as, or in, a single device or apparatus, such as a workstation, e.g., laptop or desktop-based, or a server. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA). In general, each functional unit of the system may be implemented in the form of a circuit. It is noted that the system 100 may also be implemented in a distributed manner, e.g., involving different devices or apparatuses, such as distributed servers, e.g., in the form of cloud computing.

Fig. 2 shows a system 200 for using the trained model with input data and for using the metadata to determine whether the input data conforms to the numerical characteristic of the training data of the trained model. The system 200 may comprise a data interface 220 and a processor subsystem 240 which may internally communicate via data communication 224. The processor subsystem 240 may be configured to, during operation of the system 200 and using the data interface 220, access the model data 050 and the metadata 060 as described with reference to Fig. 1, as well as input data 040 to which the trained model is to be applied. For example, as also shown in Fig. 2, the data interface 220 may provide access 222 to an external data storage 022 which comprises said data 040-060. Alternatively, the data 040-060 may be accessed from an internal data storage. Alternatively, the data 040-060 may be received via a network. In general, the data interface 220 may take various forms, such as a network interface to a local or wide area network, e.g., the Internet, a storage interface to an internal or external data storage, etc. The data storage 022 may take any known and suitable form.

The processor subsystem 240 may be further configured to, during operation of the system 200, apply the trained model 050 to the input data 040 to obtain a further intermediate output of the trained model, determine whether the input data 040 conforms to the numerical characteristic of the training data of the trained model based on the further intermediate output, and if the input data is determined not to conform to the numerical characteristic, generate an output signal 242 indicative of said non-conformance. As an optional component, the system 200 may comprise a display output interface 260 or any other type of output interface for outputting the output signal 242 to a rendering device, such as a display 280. For example, the display output interface 260 may generate display data 262 for the display 280 which causes the display 280 to render the output signal in a sensory perceptible manner, e.g., as an on-screen warning dialog box 282.

In general, the system 200 may be embodied as, or in, a single device or apparatus, such as a workstation, e.g., laptop or desktop-based, or a mobile device. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA). In general, each functional unit of the system may be implemented in the form of a circuit. It is noted that the system 200 may also be implemented in a distributed manner, e.g., involving different devices or apparatuses, such as the client and server of a client-server implementation.

Fig. 3 shows a detailed yet non-limiting example of how a numerical characteristic may be determined for a trained model, for example, by the system 100 of Fig. 1. In this example, the trained model is a trained neural network, and more specifically a Deep Neural Network (DNN) model trained by deep learning (DL). For that purpose, deep learning techniques may be used, as known per se, which may be based on error function minimization, gradient descent methods, error back-propagation and (specifically in deep learning) mini-batch optimization, etc. The aforementioned techniques may be considered textbook methods, but other suitable techniques may be used as well. This trained model 320 may take the form of a deep neural network **M** with a suitable network architecture and a set of hidden units ***u*.** Fig. 3 further shows training data **T** which may be used for several purposes. Firstly, the training data **T** may be used to train the deep learning model **M.** Secondly, the training data **T** may be used to estimate a Generative Adversarial Network **(GAN)** 310 (Goodfellow, 2014, see reference section) which may be used to generate so-termed 'out-of-spec' samples which are close to the distribution of **T,** but do not actually derive from this distribution, e.g., are not actual observations like the training data **T.** Alternatively, the out-of-spec data may be obtained from another source, e.g., from actual observations, e.g., from acquired images which are considered to represent out-of-spec images. The trained model **M** may be applied to both the out-of-spec data and to the training data **T.** This may result in the generation of model outputs, including all intermediate model outputs **U.**

Two probability distributions, namely **P(U|out-of-spec)** 335 and **P(U|T)** 330 may describe the sampling probability of selected intermediate model unit outputs **{u}.** Such selected intermediate model units may for example correspond to selected hidden units of the trained model **M,** with their outputs corresponding to their activation values. Such hidden units may also be simply referred to as units and their activation values as unit activations or simply as intermediate model output. A normal distribution of the activation values of these units **{u}** may be characterized in terms of means **µᵢ** and covariance **σ_{ïj},** where the indexes **i** and **j** correspond to all units in **{u}** that have been selected. Note that **{u}** may be a subset of the complete set of hidden units of the model **M.** In some cases, a large number of units in **{u}** may necessitate a simple distribution, where **σᵢⱼ** = 0 **if i ≠ j.** However, in general, σᵢⱼ may be selected with or without off-diagonal non-zero values. The selection of units in the distribution and which σᵢⱼ are non-zero may be represented in Fig. 3 as **{u}, {σᵢⱼ}** 360.

Given selection **{u}, {σᵢⱼ},** the training data **T** and the (GAN-generated) out-of-spec data, the distributions **P(U|out-of-spec)** and **P(U|T)** may be estimated, e.g., by simply taking means and (co-)variances of the unit activations that result from applying the trained model M to the data objects of the training data T and the out-of-spec data, respectively. A selection of **{u}, {σᵢⱼ}** may be preferred where the difference between the two distributions is maximal, as a probability distribution is desired that can distinguish samples that come from the training data **T,** or from an identical source as the training data, from the aforementioned type and other types of out-of-spec data. This difference may be expressed by the Kullback-Leibler distance 340, cross-entropy, mutual information or other measures as known per se.

To maximize the difference between the distributions **P(U|out-of-spec)** and **P(U|T),** a combinatorial optimization method 350 may be used, e.g., from the family of genetic algorithms ('GenAl'), integer linear programming, etc. Alternatively, other combinatorial optimization methods as known per se in the field may be used.

As a result, a **P(U|T)** and **P(U|out-of-spec)** may be obtained which may be optimal, or at least well-suited, to distinguish samples from the training data **T** from out-of-spec data samples. One may consider to use **P(U|out-of-spec)** to estimate whether a new input data object is to be considered out-of-spec. However, it may be preferable to use **P(U|T)** as an in-spec detector instead, as one may aim for the training data **T** to be a complete description of in-spec data, whereas for the out-of-spec data, it may be difficult, if not impossible, to determine whether all out-of-spec situations have been covered. As such, **P(U|T)** may be encoded as metadata to the deep learning model **M.** When using the deep learning model **M** on new input data, one may estimate whether an input data object, such as an image, is to be considered in-spec or out-of-spec by using the input data object as input to the deep learning model **M** to estimate **U_{new}** and by assigning an in-spec probability **P(U_{new} IT)** to the input data object, using the distribution function **P(U|T)** provided by the metadata. For example, if the in-spec probability **P(U_{new} |T)** exceeds a pre-set threshold θ, the input data object may be considered in-spec and else out-of-spec.

It is noted that when the trained model is applied to images as training data, as out-of-spec data or as 'new' input data, each image may, when input to the trained model, result in a vector in which each element I may correspond to the activation value of hidden unit i. This vector may be seen as one sample drawn from an n-dimensional distribution (n being the number of hidden units considered / the length of the vector), which may for example be described by a mean **p** (of length n) and variance matrix **a** (n x n). During training, multiple of such images may define the distribution, e.g., by mean **µ** and variance **σ,** which may be calculated directly from the collection of activation vectors. The above may also apply to other training data which comprises multiple training data objects, mutatis mutandis.

With further reference to the out-of-spec data: instead or in addition to this data being generated by a GAN 310, the data may be acquired from elsewhere, e.g., in a same or similar manner as the training data **T** is acquired, e.g., from a medical scan apparatus. However, while the training data **T** may be annotated in order to perform supervised training of the model **M,** the out-of-spec data may not need to be annotated as the model **M** is not trained based on this data. For example, if the trained model is used for medical image classification, the out-of-spec data may be obtained by filtering logging information of medical scan apparatuses for known out-of-spec usages, and by using the image(s) acquired by such out-of-spec usages as the out-of-spec data. For example, machine operation logs of a CT scanner or a pathology image scanner, and possible logged protocols, e.g., denoting the preparation of a pathology tissue sample, may be filtered. Such out-of-spec data may also be gathered in similar ways in non-image based medical contexts, e.g., in the case of EEG or ECG data, or in general in non-medical contexts. Thereby, out-of-spec data objects may be generated that contribute to obtaining a **P(U|T)** that describes the in-spec usage more precisely, e.g., to draw sharp borders between in- and out-of-spec data. With continued reference to **Fig. 3****,** the out-of-spec data may be used as input to the deep learning model **M** at the location/instead of the **GAN.**

In general, the **GAN** 310 may be used to generate out-of-spec data in a more general manner, as it does not rely on having to acquire out-of-spec data. Using the GAN generator directly may not be optimal, as the generator may be of such high quality that a human may have difficulty distinguishing generated from real data. However, it is known to apply a GAN to produce so-called 'negative samples', which may be used as out-of-spec samples in the present context. For example, this is known from Yu 2017, Dai 2017, Wang 2018, and Zheng 2018.

The generating of such negative samples may involve a trade-off between being outside of the 'in-spec' characteristics, e.g., the characteristics of the training data **T,** but close enough to these characteristics so as to allow a sharp boundary to be defined between in- and out-of-spec data. Finally, a mixture of GAN-generated out-of-spec data and otherwise acquired out-of-spec data may be used as well.

Although the above describes the numerical characteristic to be a probability distribution, various other types of numerical characterizations may be used as well, as known per se from field of statistics. Moreover, if a probability distribution is used as numerical characterization, any known and suitable type, presentation, or way of calculating the probability distribution may be used.

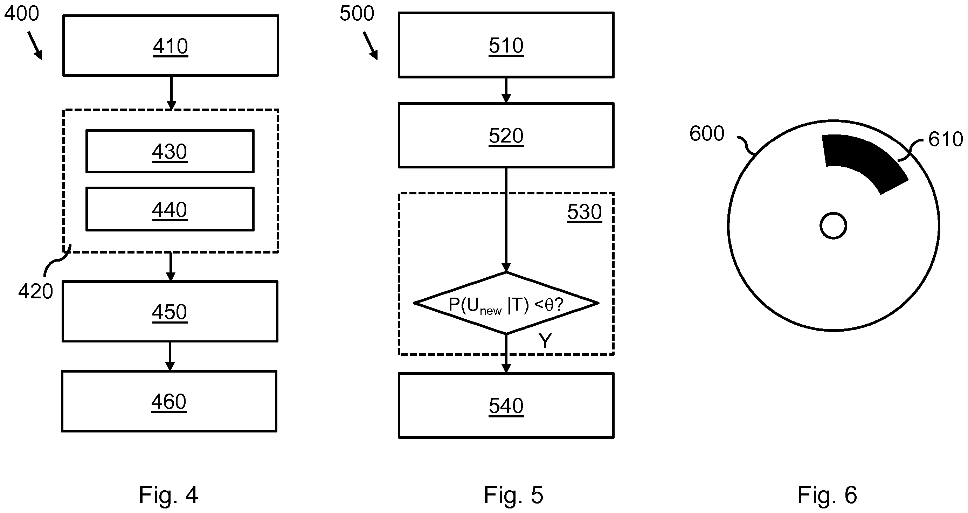

**Fig. 4** shows a block-diagram of computer-implemented method 400 for processing a trained model. The method 400 may correspond to an operation of the system 100 of Fig. 1. However, this is not a limitation, in that the method 400 may also be performed using another system, apparatus or device.

The method 400 may comprise, in an operation titled "ACCESSING MODEL DATA, TRAINING DATA", accessing 410 model data representing a trained model, and training data on which the trained model is trained. The method 400 may further comprise, in an operation titled "CHARACTERIZING TRAINING DATA", characterizing 420 the training data by, in an operation titled "APPLYING TRAINED MODEL TO TRAINING DATA", applying 430 the trained model to the training data to obtain intermediate output of the trained model, and in an operation titled "DETERMINING NUMERICAL CHARACTERISTIC", determining 440 the numerical characteristic based on the intermediate output of the trained model. The method 400 may further comprise, in an operation titled "ENCODING NUMERICAL CHARACTERISTIC AS METADATA", encoding 450 the numerical characteristic as metadata, and in an operation titled "ASSOCIATING METADATA WITH MODEL DATA", associating 460 the metadata with the model data to enable an entity applying the trained model to input data to determine whether the input data conforms to the numerical characteristic of the training data of the trained model.

**Fig. 5** shows a block-diagram of computer-implemented method 500 for using a trained model. The method 500 may correspond to an operation of the system 200 of Fig. 2. However, this is not a limitation, in that the method 500 may also be performed using another system, apparatus or device.

The method 500 may comprise, in an operation titled "ACCESSING MODEL DATA, METADATA, INPUT DATA", accessing 510 model data representing a trained model having been trained on training data, metadata associated with the model data and comprising a numerical characteristic, wherein the numerical characteristic is determined based on an intermediate output of the trained model when applied to the training data, and input data to which the trained model is to be applied. The method 500 may further comprise, in an operation titled "APPLYING TRAINED MODEL TO INPUT DATA", applying 520 the trained model to the input data to obtain a further intermediate output of the trained model. The method 500 may further comprise, in an operation titled "DETERMINING CONFORMANCE OF INPUT DATA", determining 530 whether the input data conforms to the numerical characteristic of the training data of the trained model based on the further intermediate output. Such determining of (non)conformance may, for example, involve comparing a probability **P(U_{new} |T)** to a pre-set threshold θ, as explained earlier with Fig. 3 and elsewhere. The method 500 may further comprise, in an operation titled "GENERATING OUTPUT SIGNAL INDICATIVE OF NON-CONFORMANCE", if the input data is determined not to conform to the numerical characteristic, generating 540 an output signal indicative of said non-conformance.

It will be appreciated that, in general, the operations of method 400 of Fig. 4 and/or method 500 of Fig. 5 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 6****,** instructions for the computer, e.g., executable code, may be stored on a computer readable medium 600, e.g., in the form of a series 610 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 6 shows an optical disc 600. Alternatively, the computer readable medium 600 may comprise transitory or non-transitory data 610 representing metadata as described elsewhere in this specification.

It will be appreciated that while examples are given within the medical domain, the techniques described in this specification may also be applied to various other application areas, such as autonomous driving where the input data may be sensor data and the trained model may be trained to take autonomous driving decisions based on the sensor data. Accordingly, a user may be warned if the sensor data does not conform to the characteristics of the sensor data on which the trained model was trained, for example, when the trained model was trained on summer driving conditions but used in winter driving conditions. In general, the trained model may be trained and used for classification or regression of input data.

For example, artificial intelligence methods may be applied in traffic or crowd analysis, for example counting and tracking people and vehicles, and may be based on machine learnable modes which are trained on training data. Changing conditions such as meteorological conditions (snow, fog, pollen) or operating conditions (extreme heat or cold) may influence the images and lead to poor Al performance. Out-of-spec detection can warn the human user to intervene.

In data traffic optimization, advanced machine-learning algorithms may take large-scale and highly granular network data as inputs to generate precise demand forecasts for each node in the network and detect inter-temporal patterns in network traffic and utilization, which may again be based on machine learnable models trained on training data. The improved traffic and demand prediction may enable more accurate assessment of network capacity requirements and reduce the need for resource over-provision. Use of the network may however change over time or be disrupted altogether (e.g. DDOS attacks). Early detection of such disruption or changes in network traffic, in the form of an out-of-spec detection for a given trained model, allow organizations to take proactive actions to ensure network performance.

### References

Diederik P Kingma, M. W. (2013). Auto-Encoding Variational Bayes. Arxiv, arXiv:1312.6114 .

Goodfellow, 1. J. (2014). Generative Adversarial Networks. arXiv, arXiv:1406.2661.

Yu, Y et al (2017). Open-Category Classification by Adversarial Sample Generation. Proceedings of the Twenty-Sixth International Joint Conference on Artificial Intelligence (IJCAI-17)

Dai, Z. et al (2017). Good Semi-supervised Learning That Requires a Bad GAN. 31st Conference on Neural Information Processing Systems (NIPS 2017), Long Beach, CA, USA.

Wang P. et al (2018). Incorporating GAN for Negative Sampling in Knowledge Representation Learning. AAAI Publications, Thirty-Second AAAI Conference on Artificial Intelligence

Zheng, P. et al (2018). One-Class Adversarial Nets for Fraud Detection. arXiv:1803.01798v2 [cs.LG] 5 Jun 2018.

In the claims, any reference signs placed between parentheses shall not be construed as limiting the claim. Use of the verb "comprise" and its conjugations does not exclude the presence of elements or stages other than those stated in a claim. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware.

## Claims

1. A system (100) for processing a trained model configured for image classification or image segmentation, comprising:
- a data interface (120) for accessing:
- model data (050) representing a trained model trained for image classification or image segmentation, wherein the trained model is a trained neural network, and
- training data (030) acquired from a medical scan apparatus on which the trained model is trained, wherein the training data (030) comprises multiple images;
- a processor subsystem (140) configured to:
- characterize the training data by:
- applying the trained model to the training data to obtain intermediate output of the trained model, wherein the intermediate output comprises activation values of a subset of hidden units of the trained neural network, and
- determining a numerical characteristic which is descriptive of the training data based on the intermediate output of the trained model;
- encode the numerical characteristic as metadata (060); and
- associate the metadata with the model data to enable an entity applying the trained model to input data acquired in a similar manner as the training data and thereby obtaining further intermediate output of the trained model to determine whether the input data conforms to the numerical characteristic of the training data of the trained model based on the further intermediate output.

2. The system (100) according to claim 1, wherein the training data (030) comprises multiple training data objects, and wherein the processor subsystem (140) is configured to:
- apply the trained model to individual ones of the multiple training data objects to obtain multiple sets of activation values; and
- determine the numerical characteristic as a probability distribution of the multiple sets of activation values.

3. The system (100) according to claim 2, wherein the processor subsystem (140) is configured to:
- obtain out-of-spec data comprising multiple out-of-spec data objects which are different from, and have characteristics which do not conform to the characteristics of, the multiple training data objects;
- apply the trained neural network to individual ones of the multiple out-of-spec data objects to obtain further multiple sets of activation values; and
- select the subset of hidden units to establish a difference, or to increase or maximize the difference, between a) the probability distribution of the multiple sets of activation values and b) a probability distribution of the further multiple sets of activation values.

4. The system (100) according to claim 3, wherein the processor subsystem (140) is configured to select the subset of hidden units by a combinatorial optimization method which optimizes the difference between a) the probability distribution of the multiple sets of activation values and b) the probability distribution of the further multiple sets of activation values, as a function of selected hidden units.

5. The system (100) according to claim 4, wherein the processor subsystem (140) is configured to express the difference as or based on at least one of the group of:
- a Kullback-Leibler divergence measure,
- a cross entropy measure, and
- a mutual information measure.

6. The system (100) according to any one of claims 3 to 5, wherein the processor subsystem (140) is configured to:
- use a generator part of a generative adversarial network to generate negative samples on the basis of the training data;
- generate the out-of-spec data from the negative samples.

7. The system (100) according to any one of claims 1 to 6, wherein the processor subsystem (140) is configured to generate the model data (050) by training a model using the training data (030), thereby obtaining the trained model.

8. A computer-implemented method (400) of processing a trained model configured for image classification or image segmentation, comprising:
- accessing (410):
- model data representing a trained model trained for image classification or image segmentation, wherein the trained model is a trained neural network, and
- training data acquired from a medical scan apparatus on which the trained model is trained, wherein the training data (030) comprises multiple images;
- characterizing (420) the training data by:
- applying (430) the trained model to the training data to obtain intermediate output of the trained model, wherein the intermediate output comprises activation values of a subset of hidden units of the trained neural network, and
- determining (440) the numerical characteristic which is descriptive of the training data based on the intermediate output of the trained model;
- encoding (450) the numerical characteristic as metadata; and
- associating (460) the metadata with the model data to enable an entity applying the trained model to input data acquired in a similar manner as the training data and thereby obtaining further intermediate output of the trained model to determine whether the input data conforms to the numerical characteristic of the training data of the trained model based on the further intermediate output.

9. A system (200) for using a trained model configured for image classification or image segmentation, comprising:
- a data interface (220) for accessing:
- model data (050) representing a trained model having been trained on training data acquired from a medical scan apparatus, wherein the trained model is a trained neural network trained for image classification or image segmentation,
- metadata (060) associated with the model data and comprising a numerical characteristic which is descriptive of the training data, wherein the numerical characteristic is determined based on an intermediate output of the trained model when applied to the training data, wherein the intermediate output comprises activation values of a subset of hidden units of the trained neural network, and
- input data (040) to which the trained model is to be applied, wherein the input data is acquired in a similar manner as the training data;
- a processor subsystem (240) configured to:
- apply the trained model to the input data to obtain a further intermediate output of the trained model;
- determine whether the input data conforms to the numerical characteristic of the training data of the trained model based on the further intermediate output; and
- if the input data is determined not to conform to the numerical characteristic, generate an output signal (242) indicative of said non-conformance.

10. The system (200) according to claim 9, further comprising an output interface (260) for outputting the output signal (242) to a rendering device (280) for rendering the output signal in a sensory perceptible manner to a user.

11. The system (200) according to claim 9 or 10, , wherein the numerical characteristic is a probability distribution obtained from multiple sets of activation values of a subset of hidden units of the trained neural network, wherein the multiple sets of activation values are obtained by applying the trained model to the training data, wherein the further intermediate output of the trained model comprises a further set of activation values of the subset of hidden units, and wherein the processor subsystem (240) is configured to:
- determine a probability of the further set of activation values based on the probability distribution; and
- determine whether the input data conforms to the numerical characteristic of the training data of the trained model as a function of the probability.

12. A computer-implemented method (500) of using a trained model configured for image classification or image segmentation,, comprising:
- accessing (510):
- model data representing a trained model having been trained on training data acquired from a medical scan apparatus, wherein the trained model is a trained neural network trained for image classification or image segmentation,
- metadata associated with the model data and comprising a numerical characteristic which is descriptive of the training data, wherein the numerical characteristic is determined based on an intermediate output of the trained model when applied to the training data, wherein the intermediate output comprises activation values of a subset of hidden units of the trained neural network, and
- input data to which the trained model is to be applied, wherein the input data is acquired in a similar manner as the training data;
- applying (520) the trained model to the input data to obtain a further intermediate output of the trained model;
- determining (530) whether the input data conforms to the numerical characteristic of the training data of the trained model based on the further intermediate output; and
- if the input data is determined not to conform to the numerical characteristic, generating (540) an output signal indicative of said non-conformance.

13. A computer-readable medium (600) comprising instructions, which when executed by a computer, cause the computer to carry out the steps of the computer-implemented method according to claim 8 or 12.

## Patentansprüche

1. System (100) zur Verarbeitung eines trainierten Modells, das zur Bildklassifizierung oder Bildsegmentierung konfiguriert ist, umfassend:
- eine Datenschnittstelle (120) für den Zugriff auf:
- Modelldaten (050), die ein trainiertes Modell darstellen, das für die Bildklassifizierung oder Bildsegmentierung trainiert wurde, wobei das trainierte Modell ein trainiertes neuronales Netzwerk ist, und
- Trainingsdaten (030), die von einer medizinischen Scan-Einrichtung erfasst werden, auf der das trainierte Modell trainiert wird, wobei die Trainingsdaten (030) mehrere Bilder umfassen;
ein Prozessor-Subsystem (140), konfiguriert zum:
- Charakterisieren der Trainingsdaten durch:
- Anwenden des trainierten Modells auf die Trainingsdaten, um eine Zwischenausgabe des trainierten Modells zu erhalten, wobei die Zwischenausgabe Aktivierungswerte einer Teilmenge von versteckten Einheiten des trainierten neuronalen Netzwerks umfasst, und
- Bestimmen einer Kenngröße, die die Trainingsdaten beschreibt, auf der Grundlage der Zwischenausgabe des trainierten Modells;
- Codieren der Kenngröße als Metadaten (060); und
- Zuordnen der Metadaten zu den Modelldaten, um es einer Instanz zu ermöglichen, das trainierte Modell auf Eingabedaten anzuwenden, die in ähnlicher Weise wie die Trainingsdaten gewonnen wurden, und dadurch eine weitere Zwischenausgabe des trainierten Modells zu erhalten, um auf der Grundlage der weiteren Zwischenausgabe zu bestimmen, ob die Eingabedaten mit der Kenngröße der Trainingsdaten des trainierten Modells übereinstimmen.

2. System (100) nach Anspruch 1, wobei die Trainingsdaten (030) mehrere Trainingsdatenobjekte umfassen, und wobei das Prozessor-Subsystem (140) konfiguriert ist zum:
- Anwenden des trainierten Modells auf einzelne der mehreren Trainingsdatenobjekte, um mehrere Sätze von Aktivierungswerten zu erhalten; und
- Bestimmen der Kenngröße als Wahrscheinlichkeitsverteilung der mehreren Sätze von Aktivierungswerten.

3. System (100) nach Anspruch 2, wobei das Prozessor-Subsystem (140) konfiguriert ist zum:
- Erhalten von nicht normgerechten Daten, die mehrere nicht normgerechte Datenobjekte umfassen, die sich von den mehreren Trainingsdatenobjekten unterscheiden und Merkmale aufweisen, die nicht mit den Merkmalen dieser Objekte übereinstimmen;
- Anwenden des trainierten neuronalen Netzwerks auf einzelne der mehreren nicht normgerechten Datenobjekte, um weitere mehrere Sätze von Aktivierungswerten zu erhalten; und
- Auswählen der Teilmenge versteckter Einheiten, um eine Differenz zwischen a) der Wahrscheinlichkeitsverteilung der mehreren Sätze von Aktivierungswerten und b) einer Wahrscheinlichkeitsverteilung der weiteren mehreren Sätze von Aktivierungswerten herzustellen oder die Differenz zu vergrößern oder zu maximieren.

4. System (100) nach Anspruch 3, wobei das Prozessor-Subsystem (140) so konfiguriert ist, dass es die Teilmenge der versteckten Einheiten durch ein kombinatorisches Optimierungsverfahren auswählt, das die Differenz zwischen a) der Wahrscheinlichkeitsverteilung der mehreren Sätze von Aktivierungswerten und b) der Wahrscheinlichkeitsverteilung der weiteren mehreren Sätze von Aktivierungswerten in Abhängigkeit von ausgewählten versteckten Einheiten optimiert.

5. System (100) nach Anspruch 4, wobei das Prozessor-Subsystem (140) so konfiguriert ist, dass es die Differenz als oder auf der Grundlage von mindestens einem ausdrückt von:
- einem Kullback-Leibler-Divergenzmaß,
- einem Kreuzentropiemaß und
- einem gegenseitigen Informationsmaß.

6. System (100) nach einem der Ansprüche 3 bis 5, wobei das Prozessor-Subsystem (140) konfiguriert ist zum:
- Verwenden eines Generators, der Teil eines generativen adversen Netzwerks ist, um auf der Grundlage der Trainingsdaten negative Muster zu erzeugen;
- Erzeugen der nicht normgerechten Daten aus den negativen Proben.

7. System (100) nach einem der Ansprüche 1 bis 6, wobei das Prozessor-Subsystem (140) so konfiguriert ist, dass es die Modelldaten (050) durch Training eines Modells unter Verwendung der Trainingsdaten (030) erzeugt, wodurch das trainierte Modell erhalten wird.

8. Computerimplementiertes Verfahren (400) zur Verarbeitung eines trainierten Modells, das zur Bildklassifizierung oder Bildsegmentierung konfiguriert ist, umfassend:
- Zugreifen (410):
- Modelldaten, die ein trainiertes Modell darstellen, das für die Bildklassifizierung oder Bildsegmentierung trainiert wurde, wobei das trainierte Modell ein trainiertes neuronales Netzwerk ist, und
- Trainingsdaten, die von einer medizinischen Scan-Einrichtung erfasst werden, auf der das trainierte Modell trainiert wird, wobei die Trainingsdaten (030) mehrere Bilder umfassen;
- Charakterisieren (420) der Trainingsdaten durch:
- Anwenden (430) des trainierten Modells auf die Trainingsdaten, um eine Zwischenausgabe des trainierten Modells zu erhalten, wobei die Zwischenausgabe Aktivierungswerte einer Teilmenge von versteckten Einheiten des trainierten neuronalen Netzwerks umfasst, und
- Bestimmen (440) der Kenngröße, die die Trainingsdaten beschreibt, auf der Grundlage der Zwischenausgabe des trainierten Modells;
- Codieren (450) der Kenngröße als Metadaten; und
- Zuordnen (460) der Metadaten zu den Modelldaten, um es einer Instanz zu ermöglichen, das trainierte Modell auf Eingabedaten anzuwenden, die in ähnlicher Weise wie die Trainingsdaten gewonnen wurden, und dadurch eine weitere Zwischenausgabe des trainierten Modells zu erhalten, um auf der Grundlage der weiteren Zwischenausgabe zu bestimmen, ob die Eingabedaten mit der Kenngröße der Trainingsdaten des trainierten Modells übereinstimmen.

9. System (200) zur Verwendung eines trainierten Modells, das zur Bildklassifizierung oder Bildsegmentierung konfiguriert ist, umfassend:
- eine Datenschnittstelle (220) für den Zugriff auf:
- Modelldaten (050), die ein trainiertes Modell darstellen, das auf Trainingsdaten trainiert wurde, die von einer medizinischen Scan-Einrichtung erfasst wurden, wobei das trainierte Modell ein trainiertes neuronales Netzwerk ist, das zur Bildklassifizierung oder Bildsegmentierung trainiert wurde,
- Metadaten (060), die den Modelldaten zugeordnet sind und eine Kenngröße umfassen, die die Trainingsdaten beschreibt, wobei die Kenngröße auf der Grundlage einer Zwischenausgabe des trainierten Modells bestimmt wird, wenn sie auf die Trainingsdaten angewendet wird, wobei die Zwischenausgabe Aktivierungswerte einer Teilmenge von versteckten Einheiten des trainierten neuronalen Netzwerks umfasst, und
- Eingabedaten (040), auf die das trainierte Modell angewendet werden soll, wobei die Eingabedaten auf ähnliche Weise wie die Trainingsdaten erfasst werden;
ein Prozessor-Subsystem (240), konfiguriert zum:
- Anwenden des trainierten Modells auf die Eingabedaten, um eine weitere Zwischenausgabe des trainierten Modells zu erhalten;
- Bestimmen, ob die Eingangsdaten mit der Kenngröße der Trainingsdaten des trainierten Modells übereinstimmen, auf der Grundlage der weiteren Zwischenausgabe; und
- wenn festgestellt wird, dass die Eingangsdaten nicht mit der Kenngröße übereinstimmen, Erzeugen eines Ausgangssignals (242), das die Nichtübereinstimmung anzeigt.

10. System (200) nach Anspruch 9, weiter umfassend eine Ausgabeschnittstelle (260) zur Ausgabe des Ausgangssignals (242) an eine Render-Vorrichtung (280) zum Rendern des Ausgangssignals in einer sensorisch wahrnehmbaren Weise für einen Benutzer.

11. System (200) nach Anspruch 9 oder 10, wobei die Kenngröße eine Wahrscheinlichkeitsverteilung ist, die aus mehreren Sätzen von Aktivierungswerten einer Teilmenge von versteckten Einheiten des trainierten neuronalen Netzwerks erhalten wird, wobei die mehreren Sätze von Aktivierungswerten durch Anwenden des trainierten Modells auf die Trainingsdaten erhalten werden, wobei die weitere Zwischenausgabe des trainierten Modells einen weiteren Satz von Aktivierungswerten der Teilmenge von versteckten Einheiten umfasst, und wobei das Prozessor-Subsystem (240) konfiguriert ist zum:
- Bestimmen einer Wahrscheinlichkeit des weiteren Satzes von Aktivierungswerten auf der Grundlage der Wahrscheinlichkeitsverteilung; und
- Bestimmen, ob die Eingabedaten mit der Kenngröße der Trainingsdaten des trainierten Modells in Abhängigkeit von der Wahrscheinlichkeit übereinstimmen.

12. Computerimplementiertes Verfahren (500) zur Verwendung eines trainierten Modells, das zur Bildklassifizierung oder Bildsegmentierung konfiguriert ist, umfassend:
- Zugreifen (510):
- Modelldaten, die ein trainiertes Modell darstellen, das auf Trainingsdaten trainiert wurde, die von einer medizinischen Scan-Einrichtung erfasst wurden, wobei das trainierte Modell ein trainiertes neuronales Netzwerk ist, das zur Bildklassifizierung oder Bildsegmentierung trainiert wurde,
- Metadaten, die den Modelldaten zugeordnet sind und eine Kenngröße umfassen, die die Trainingsdaten beschreibt, wobei die Kenngröße auf der Grundlage einer Zwischenausgabe des trainierten Modells bestimmt wird, wenn sie auf die Trainingsdaten angewendet wird, wobei die Zwischenausgabe Aktivierungswerte einer Teilmenge von versteckten Einheiten des trainierten neuronalen Netzwerks umfasst, und
- Eingabedaten, auf die das trainierte Modell angewendet werden soll, wobei die Eingabedaten auf ähnliche Weise wie die Trainingsdaten gewonnen werden;
- Anwenden (520) des trainierten Modells auf die Eingabedaten, um eine weitere Zwischenausgabe des trainierten Modells zu erhalten;
- Bestimmen (530), ob die Eingangsdaten mit der Kenngröße der Trainingsdaten des trainierten Modells übereinstimmen, auf der Grundlage der weiteren Zwischenausgabe; und
- wenn festgestellt wird, dass die Eingangsdaten nicht mit der Kenngröße übereinstimmen, Erzeugen (540) eines Ausgangssignals, das die Nichtübereinstimmung anzeigt.

13. Computerlesbares Medium (600), das Anweisungen umfasst, die, wenn sie von einem Computer ausgeführt werden, den Computer veranlassen, die Schritte des computerimplementierten Verfahrens nach Anspruch 8 oder 12 auszuführen.

## Revendications

1. Système (100) de traitement d'un modèle entraîné configuré pour la classification d'images ou la segmentation d'images, comprenant :
- une interface de données (120) permettant d'accéder à :
- des données de modèle (050) représentant un modèle entraîné qui est entraîné pour la classification d'images ou la segmentation d'images, dans lequel le modèle entraîné est un réseau neuronal entraîné, et
- des données d'entraînement (030) acquises à partir d'un appareil de balayage médical sur lequel le modèle entraîné est entraîné, dans lequel les données d'entraînement (030) comprennent de multiples images ;
- un sous-système de processeur (140) configuré pour :
- caractériser les données d'entraînement par :
- l'application du modèle entraîné aux données d'entraînement pour obtenir une sortie intermédiaire du modèle entraîné, dans lequel la sortie intermédiaire comprend des valeurs d'activation d'un sous-ensemble d'unités cachées du réseau neuronal entraîné, et
- la détermination d'une caractéristique numérique qui est descriptive des données d'entraînement sur la base de la sortie intermédiaire du modèle entraîné ;
- coder la caractéristique numérique sous forme de métadonnées (060) ; et
- associer les métadonnées aux données de modèle pour permettre à une entité d'appliquer le modèle entraîné à des données d'entrée acquises de manière similaire aux données d'entraînement et d'obtenir ainsi une sortie intermédiaire supplémentaire du modèle entraîné pour déterminer si les données d'entrée sont conformes à la caractéristique numérique des données d'entraînement du modèle entraîné sur la base de la sortie intermédiaire supplémentaire.

2. Système (100) selon la revendication 1, dans lequel les données d'entraînement (030) comprennent de multiples objets de données d'entraînement, et dans lequel le sous-système de processeur (140) est configuré pour :
- appliquer le modèle entraîné à des objets individuels parmi les multiples objets de données d'entraînement pour obtenir de multiples ensembles de valeurs d'activation ; et
- déterminer la caractéristique numérique sous forme d'une distribution de probabilité des multiples ensembles de valeurs d'activation.

3. Système (100) selon la revendication 2, dans lequel le sous-système de processeur (140) est configurée pour :
- obtenir des données hors spécifications comprenant de multiples objets de données hors spécifications qui sont différents des multiples objets de données d'entraînement et présentent des caractéristiques qui ne sont pas conformes aux caractéristiques de ces derniers ;
- appliquer le réseau neuronal entraîné à des objets de données hors spécifications individuels parmi les multiples objets de données hors spécifications pour obtenir de multiples ensembles de valeurs d'activation supplémentaires ; et
- sélectionner le sous-ensemble d'unités cachées pour établir une différence, ou pour augmenter ou maximiser la différence, entre a) la distribution de probabilité des multiples ensembles de valeurs d'activation et b) une distribution de probabilité des multiples ensembles de valeurs d'activation supplémentaires.

4. Système (100) selon la revendication 3, dans lequel le sous-système de processeur (140) est configuré pour sélectionner le sous-ensemble d'unités cachées par un procédé d'optimisation combinatoire qui optimise la différence entre a) la distribution de probabilité des multiples ensembles de valeurs d'activation et b) la distribution de probabilité des multiples ensembles de valeurs d'activation supplémentaires, en fonction des unités cachées sélectionnées.

5. Système (100) selon la revendication 4, dans lequel le sous-système de processeur (140) est configuré pour exprimer la différence sous la forme ou sur la base d'au moins l'une du groupe suivant :
- une mesure de divergence de Kullback-Leibler,
- une mesure d'entropie croisée, et
- une mesure d'information mutuelle.

6. Système (100) selon l'une quelconque des revendications 3 à 5, dans lequel le sous-système de processeur (140) est configuré pour :
- utiliser une partie génératrice d'un réseau antagoniste génératif pour générer des échantillons négatifs sur la base des données d'entraînement ;
- générer les données hors spécifications à partir des échantillons négatifs.

7. Système (100) selon l'une quelconque des revendications 1 à 6, dans lequel le sous-système de processeur (140) est configuré pour générer les données de modèle (050) par l'entraînement d'un modèle à l'aide des données d'entraînement (030), en obtenant ainsi le modèle entraîné.

8. Procédé mis en œuvre par ordinateur (400) de traitement d'un modèle entraîné configuré pour la classification d'images ou la segmentation d'images, comprenant :
- l'accès (410) à :
- des données de modèle représentant un modèle entraîné qui est entraîné pour la classification d'images ou la segmentation d'images, dans lequel le modèle entraîné est un réseau neuronal entraîné, et
- des données d'entraînement acquises à partir d'un appareil de balayage médical sur lequel le modèle entraîné est entraîné, dans lequel les données d'entraînement (030) comprennent de multiples images ;
- la caractérisation (420) des données d'entraînement par :
- l'application (430) du modèle entraîné aux données d'entraînement pour obtenir une sortie intermédiaire du modèle entraîné, dans lequel la sortie intermédiaire comprend des valeurs d'activation d'un sous-ensemble d'unités cachées du réseau neuronal entraîné, et
- la détermination (440) de la caractéristique numérique qui est descriptive des données d'entraînement sur la base de la sortie intermédiaire du modèle entraîné ;
- le codage (450) de la caractéristique numérique sous forme de métadonnées ; et
- l'association (460) des métadonnées aux données de modèle pour permettre à une entité d'appliquer le modèle entraîné à des données d'entrée acquises de manière similaire aux données d'entraînement et d'obtenir ainsi une sortie intermédiaire supplémentaire du modèle entraîné pour déterminer si les données d'entrée sont conformes à la caractéristique numérique des données d'entraînement du modèle entraîné sur la base de la sortie intermédiaire supplémentaire.

9. Système (200) permettant d'utiliser un modèle entraîné configuré pour la classification d'images ou la segmentation d'images, comprenant :
- une interface de données (220) permettant d'accéder à :
- des données de modèle (050) représentant un modèle entraîné ayant été entraîné sur des données d'entraînement acquises à partir d'un appareil de balayage médical, dans lequel le modèle entraîné est un réseau neuronal entraîné pour la classification d'images ou la segmentation d'images,
- des métadonnées (060) associées aux données de modèle et comprenant une caractéristique numérique qui est descriptive des données d'entraînement, dans lequel la caractéristique numérique est déterminée sur la base d'une sortie intermédiaire du modèle entraîné lorsqu'elle est appliquée aux données d'entraînement, dans lequel la sortie intermédiaire comprend des valeurs d'activation d'un sous-ensemble d'unités cachées du réseau neuronal entraîné, et
- des données d'entrée (040) auxquelles le modèle entraîné doit être appliqué, dans lequel les données d'entrée sont acquises de manière similaire aux données d'entraînement ;
- un sous-système de processeur (240) configuré pour :
- appliquer le modèle entraîné aux données d'entrée pour obtenir une sortie intermédiaire supplémentaire du modèle entraîné ;
- déterminer si les données d'entrée sont conformes à la caractéristique numérique des données d'entraînement du modèle entraîné sur la base de la sortie intermédiaire supplémentaire ; et
- si les données d'entrée sont déterminées comme non conformes à la caractéristique numérique, générer un signal de sortie (242) indiquant ladite non-conformité.

10. Système (200) selon la revendication 9, comprenant en outre une interface de sortie (260) pour délivrer le signal de sortie (242) à un dispositif de restitution (280) pour restituer le signal de sortie d'une manière perceptible sensoriellement par un utilisateur.

11. Système (200) selon la revendication 9 ou 10, dans lequel la caractéristique numérique est une distribution de probabilité obtenue à partir de multiples ensembles de valeurs d'activation d'un sous-ensemble d'unités cachées du réseau neuronal entraîné, dans lequel les multiples ensembles de valeurs d'activation sont obtenus en appliquant le modèle entraîné aux données d'entraînement, dans lequel la sortie intermédiaire supplémentaire du modèle entraîné comprend un ensemble de valeurs d'activation supplémentaire du sous-ensemble d'unités cachées, et dans lequel le sous-système de processeur (240) est configuré pour :
- déterminer une probabilité de l'ensemble de valeurs d'activation supplémentaire sur la base de la distribution de probabilité ; et
- déterminer si les données d'entrée sont conformes à la caractéristique numérique des données d'entraînement du modèle entraîné en fonction de la probabilité.

12. Procédé mis en œuvre par ordinateur (500) d'utilisation d'un modèle entraîné configuré pour la classification d'images ou la segmentation d'images, comprenant :
- l'accès (510) à :
- des données de modèle représentant un modèle entraîné ayant été entraîné sur des données d'entraînement acquises à partir d'un appareil de balayage médical, dans lequel le modèle entraîné est un réseau neuronal entraîné pour la classification d'images ou la segmentation d'images,
- des métadonnées associées aux données de modèle et comprenant une caractéristique numérique qui est descriptive des données d'entraînement, dans lequel la caractéristique numérique est déterminée sur la base d'une sortie intermédiaire du modèle entraîné lorsqu'elle est appliquée aux données d'entraînement, dans lequel la sortie intermédiaire comprend des valeurs d'activation d'un sous-ensemble d'unités cachées du réseau neuronal entraîné, et
- des données d'entrée auxquelles le modèle entraîné doit être appliqué, dans lequel les données d'entrée sont acquises de manière similaire aux données d'entraînement ;
- l'application (520) du modèle entraîné aux données d'entrée pour obtenir une sortie intermédiaire supplémentaire du modèle entraîné ;
- la détermination (530) du fait que les données d'entrée sont ou non conformes à la caractéristique numérique des données d'entraînement du modèle entraîné sur la base de la sortie intermédiaire supplémentaire ; et
- si les données d'entrée sont déterminées comme non conformes à la caractéristique numérique, la génération (540) d'un signal de sortie indiquant ladite non-conformité.

13. Support lisible par ordinateur (600) comprenant des instructions qui, lorsqu'elles sont exécutées par un ordinateur, amènent l'ordinateur à réaliser les étapes du procédé mis en œuvre par ordinateur selon la revendication 8 ou 12.
